# EUROPÄISCHE PATENTSCHRIFT

(11) **EP 2 805 666 B1**
(45) Veröffentlichungstag und Bekanntmachung des Hinweises auf die Patenterteilung: **19.07.2017**
(21) Anmeldenummer: 14167715.3
(22) Anmeldetag: 09.05.2014
(51) Int. Cl.: A61B 1/00, A61B 1/002, G02B 7/02, G02B 23/24

(54) **Optikrohr für ein Endoskop, Stablinse, Kombination aus einem Optikrohr und mindestens einer Stablinse sowie Endoskop**
Optical tube for an endoscope, rod lens, combination of an optical tube and at least one rod lens and endoscope
Tube optique pour un endoscope, lentille cylindrique, combinaison d'un tube optique et d'au moins une lentille cylindrique et endoscope

(30) Priorität: 22.05.2013 DE 102013105233
(43) Veröffentlichungstag der Anmeldung: 26.11.2014
(73) Patentinhaber: Karl Storz GmbH & Co. KG, 78532 Tuttlingen (DE)
(72) Erfinder: Vogel, Dr. Walter, 78532 Tuttlingen (DE)

(56) Entgegenhaltungen:
- EP-B1- 1 320 319
- DE-A1- 19 732 991
- DE-A1-102006 041 920
- JP-A- 2012 080 944
- US-A- 5 933 287

## Beschreibung

Die vorliegende Erfindung betrifft ein Optikrohr für ein Endoskop, eine Stablinse, eine Kombination aus einem Optikrohr und mindestens einer Stablinse sowie ein Endoskop. Endoskope dienen zur Betrachtung von Hohlräumen im menschlichen oder tierischen Körper sowie in technischen Gegenständen. Ein Endoskop umfasst typischerweise einen lang erstreckten Endoskopschaft, der zur Einführung in den zu betrachtenden Hohlraum geeignet ist, sowie einen Kopf, der Anschlüsse und Bedienelemente sowie einen Okulareinblick aufweisen kann. Innerhalb des Endoskopschafts und des Kopfs ist ein optisches System zur Weiterleitung eines endoskopischen Bildes vom distalen, d.h. beobachterfernen, Ende des Endoskops zum proximalen, d.h. beobachternahen, Ende angeordnet. Das optische System umfasst hierfür insbesondere ein distal angeordnetes Objektiv zur Aufnahme des endoskopischen Bilds, einen Bildweiterleiter und ein am proximalen Ende des Endoskops angeordnetes Okular mit einem Okulareinblick zur visuellen Betrachtung des weitergeleiteten endoskopischen Bildes. Das Endoskop kann insbesondere als starres Endoskop mit einem starren Endoskopschaft ausgebildet sein und als Bildweiterleiter eine Stablinsenanordnung aufweisen, die in einem innerhalb des Endoskops angeordneten Optikrohr aufgenommen ist. Ferner kann das Endoskop zur Beleuchtung des zu beobachtenden Hohlraums eine Beleuchtungseinrichtung aufweisen sowie ggf. weitere Vorrichtungen, wie etwa Kanäle für endoskopische Arbeitsinstrumente.

Bei der Montage der optischen Bauelemente in dem Optikrohr eines starren Endoskops werden diese üblicherweise von der proximalen Seite her in der durch die Berechnung des optischen Systems vorgegebenen Reihenfolge und Anordnung in das Optikrohr eingeschoben. Insbesondere werden nacheinander das Objektiv und eine oder mehrere Stablinsen sowie ggf. eine oder mehrere Aperturblenden und/oder Abstandshalter in das Optikrohr eingeschoben. Hierfür weisen die optischen Bauelemente einen Außendurchmesser auf, der geringfügig kleiner ist als der Innendurchmesser des Optikrohrs. Mit Hilfe einer Systemfeder und einer Systemabschlussführung wird das gesamte optische System in distaler Richtung vorgespannt und dadurch gegen einen distalen Abschluss des Optikrohrs, der insbesondere durch ein Deckglas gegeben sein kann, gehalten. Hierdurch wird erreicht, dass die optischen Elemente, insbesondere die Stablinsen, in einfacher Weise im Optikrohr an ihrer vorgegebenen axialen Position montiert und bei einer Reparatur wieder entnommen werden können.

Bei ihrer Benutzung sind Endoskope erheblichen mechanischen Belastungen ausgesetzt, insbesondere Beschleunigungen bzw. Erschütterungen sowie auch Biegungen. Durch die von der Systemfeder ausgeübte Vorspannung werden die optischen Elemente dabei in Längsrichtung an ihrer vorbestimmten jeweiligen Position gehalten. In Querrichtung sind die optischen Elemente aufgrund ihres geringfügig kleineren Durchmessers jedoch gegenüber dem Optikrohr beweglich. Insbesondere können sich die Stablinsen gegeneinander und gegenüber dem Optikrohr in Querrichtung bewegen und können gegeneinander sowie gegenüber dem Optikrohr verkippen, wodurch die optischen Achsen der Stablinsen nicht mehr genau miteinander fluchten. Ferner können sich die Stablinsen um ihre Längsachse drehen, was aufgrund der fertigungsbedingten Toleranzen, denen die Stablinsen unterliegen, ebenfalls zu einer Verschlechterung der Abbildungsqualität führen kann.

Aus der nicht vorveröffentlichten Patentanmeldung DE ... (internes Aktenzeichen A12099) ist es bekannt, eine im Inneren eines Optikrohrs eines Endoskops aufgenommene Stablinse mit Hilfe einer schweißbaren Hülse an einer gewünschten Position innerhalb des Optikrohrs zu fixieren. Die Hülse kann ein ferromagnetisches Material aufweisen. Hierdurch wird eine besonders einfache Handhabung bei der Positionierung der Stablinse innerhalb des Optikrohrs ermöglicht, indem die Hülse von einem außerhalb des Optikrohrs angeordneten Magneten an die Innenseite des Optikrohrs angedrückt und sodann in der hierdurch definierten Lage mit dem Optikrohr verschweißt wird.

Aus der Ptentanmeldung DE 10 2006 041920 A1 ist ein Optikrohr bekannt, bei dem die Stablinse durch nach innen verformbare Zungen des Optikrohrs fixiert wird.

Es ist Aufgabe der vorliegenden Erfindung, ein Optikrohr für ein Endoskop, eine Stablinse, eine Kombination aus einem Optikrohr und mindestens einer Stablinse sowie ein Endoskop anzugeben, wobei auf einfache Weise eine sichere Lagerung mindestens einer Stablinse im Optikrohr erreichbar ist, und wobei insbesondere eine Dezentrierung, eine Verkippung und/oder eine Rotation der Stablinse um ihre Längsachse verhindert werden kann.

Diese Aufgabe wird durch eine Stablinse gemäß Anspruch 1, durch eine Kombination gemäß Anspruch 4 sowie durch ein Endoskop gemäß Anspruch 11 gelöst.

Vorteilhafte Weiterbildungen der Erfindung ergeben sich aus den Unteransprüchen.

Ein erfindungsgemäßes Optikrohr für ein Endoskop ist vorzugsweise langerstreckt zylindrisch geformt und zur Aufnahme einer Stablinsenanordnung eines Endoskops, die mindestens eine Stablinse umfasst, im Inneren des Optikrohrs ausgebildet. Das Optikrohr ist insbesondere ein Optikrohr eines starren Endoskops und ist zur Aufnahme des aus einer Mehrzahl von aufeinanderfolgend angeordneten Stablinsen aufgebauten Bildweiterleiters des Endoskops ausgebildet. Das Optikrohr ist vorzugsweise metallisch und besteht beispielsweise aus Edelstahl.

Erfindungsgemäß weist das Optikrohr eine sich parallel zu einer Längsachse des Optikrohrs erstreckende Magnetanordnung auf. Die Magnetanordnung des Optikrohrs ist eine Anordnung eines ferromagnetischen Materials oder umfasst zumindest ein ferromagnetisches Material und kann beispielsweise als mit dem Optikrohr verbundenes Bauteil, d.h. als Magnet, oder als auf das Optikrohr etwa als Schicht aufgebrachtes Magnetmaterial ausgebildet sein. Die Magnetanordnung ist in Querrichtung einseitig und somit nichtaxialsymmetrisch zur Längsachse des Optikrohrs angeordnet. Insbesondere ist die Magnetanordnung derart ausgebildet und angeordnet, dass bezüglich mindestens einer Längsebene, die eine Längsmittelachse des Optikrohrs enthält, das ferromagnetische Material vollständig oder zumindest überwiegend auf einer Seite des Längsebene angeordnet ist. Die Magnetanordnung kann weich- oder permanentmagnetisch ausgebildet sein. Die Magnetanordnung kann zusammenhängend ausgebildet sein oder aus mehreren, voneinander durch Unterbrechungen getrennten Segmenten oder Abschnitten bestehen.

Dadurch, dass das Optikrohr einseitig eine sich achsenparallel erstreckende Magnetanordnung aufweist, wird es ermöglicht, ferromagnetische Elemente, insbesondere mindestens eine Stablinse mit einer dieser zugeordneten Magnetanordnung innerhalb des Optikrohrs auf derjenigen Seite, die die Magnetanordnung bzw. den überwiegenden Teil des ferromagnetischen Materials der Magnetanordnung des Optikrohrs aufweist, und somit im Wesentlichen in einer achsenparallelen Anordnung auf der betreffenden Seite des Optikrohrs magnetisch zu halten. Ferner wird hierdurch eine besonders einfache Montage von derartigen Elementen innerhalb des Optikrohrs ermöglicht, wobei im Reparaturfall weiterhin eine Entnahme aus dem Optikrohr möglich bleiben kann.

Die Magnetanordnung des Optikrohrs ist vorzugsweise in Form mindestens eines achsenparallel verlaufenden Streifens ausgebildet. In besonders bevorzugter Weise verläuft der mindestens eine achsenparallele Streifen im Wesentlichen entlang der gesamten Länge des Optikrohrs, insbesondere entlang desjenigen Bereichs des Optikrohrs, der zur Aufnahme von Stablinsen bestimmt ist. Der mindestens eine Streifen kann axial in eine Mehrzahl achsenparallel angeordneter Abschnitte aufgeteilt sein. Sofern eine Mehrzahl von parallel zueinander verlaufenden achsenparallelen Streifen vorgesehen ist, können diese durch gleichfalls achsenparallel verlaufende Unterbrechungen bzw. Lücken voneinander getrennt sein oder eng aneinander anliegen. Dadurch, dass die Magnetanordnung bzw. das ferromagnetische Material in Form eines oder mehrerer parallel zur Längsachse des Optikrohrs verlaufender Streifen angeordnet ist, wird es ermöglicht, innerhalb des Optikrohr angeordnete ferromagnetische Elemente, insbesondere Stablinsen mit entsprechenden Magnetanordnungen, mit verbesserter Genauigkeit und Sicherheit in einer linearen, parallel zur Längsachse des Optikrohrs verlaufenden Anordnung innerhalb des Optikrohrs zu halten.

Gemäß einer bevorzugten Ausführungsform der Erfindung ist die Magnetanordnung auf der Außenseite des Optikrohrs angeordnet. Um eine ausreichende magnetische Wirkung auf innerhalb des Optikrohrs angeordnete magnetische Elemente zu ermöglichen, ist das Optikrohr insbesondere entsprechend dünnwandig oder aus einem nichtferromagnetischen Material ausgebildet. Dadurch, dass die Magnetanordnung sich auf der äußeren Oberfläche des Optikrohrs befindet, wird eine Einschränkung des verfügbaren Bauraums innerhalb des Optikrohrs vermieden und eine optimale Ausnutzung des innerhalb eines Endoskopschafts verfügbaren Bauraums ermöglicht. Ferner wird hierdurch die Anbringung der Magnetanordnung und somit des ferromagnetischen Materials am Optikrohr erleichtert.

Vorzugsweise ist die Magnetanordnung des Optikrohrs auf das Optikrohr aufgeklebt. Die Magnetanordnung kann beispielsweise eine teilzylindrische Form aufweisen, deren Innendurchmesser geringfügig größer als ein Außendurchmesser eines zylindrisch ausgebildeten Optikrohrs ist, so dass zwischen der Magnetanordnung und dem Optikrohr ein Klebespalt verbleibt, der zum Fixieren der Magnetanordnung auf dem Optikrohr zumindest teilweise mit Klebstoff gefüllt wird. Hierdurch wird die Anbringung der Magnetanordnung auf dem Optikrohr weiter erleichtert.

Eine erfindungsgemäße Stablinse, die insbesondere als Stablinse eines Relaislinsensystems eines Bildweiterleiters eines starren Endoskops ausgebildet ist, umfasst eine stabförmige Linseneinheit, die aus einem einzigen stabförmigen Linsenelement oder aus mehreren miteinander verkitteten Linsenelementen besteht. Die stabförmige Linseneinheit weist eine Längsachse auf, wobei eine Länge der Linseneinheit in Richtung der Längsachse größer als ein quer zur Längsachse gemessener Durchmesser ist. Die optisch wirksamen Flächen der Stablinse sind die beiden Stirnflächen der Linseneinheit sowie ggf. im Inneren einer aus mehreren Linsenelementen zusammengesetzten Linseneinheit befindliche Grenzflächen zwischen mehreren Linsenelementen. Ein oder mehrere Linsenelemente können auch als Planstab ausgebildet sein. Die stabförmige Linseneinheit weist auf ihrer Außenseite eine Umfangsfläche auf. Vorzugsweise ist die Linseneinheit rotationssymmetrisch, insbesondere zumindest abschnittsweise zylindrisch ausgebildet, wobei die Längsachse die Symmetrieachse der Linseneinheit ist. Bei einer zumindest abschnittsweise zylindrisch ausgebildeten Linseneinheit ist die Umfangsfläche die zumindest abschnittsweise zylindrische Mantelfläche der Linseneinheit.

Eine erfindungsgemäße Stablinse umfasst eine Magnetanordnung, die einseitig, d.h. quer zur Längsachse auf einer Seite der Umfangsfläche der Linseneinheit und somit nichtaxialsymmetrisch zur Längsachse der Linseneinheit angeordnet ist. Die Magnetanordnung der Stablinse ist eine Anordnung eines ferromagnetischen Materials oder umfasst zumindest ein ferromagnetisches Material und kann beispielsweise als mit der Linseneinheit der Stablinse verbundenes Bauteil, d.h. als Magnet, oder als auf die Umfangsfläche der Linseneinheit etwa als Schicht aufgebrachtes Magnetmaterial ausgebildet sein. Insbesondere ist die Magnetanordnung derart ausgebildet und angeordnet, dass bezüglich mindestens einer Längsebene, die die Symmetrieachse einer zylindrisch ausgebildeten Linseneinheit enthält, das ferromagnetische Material vollständig oder zumindest überwiegend auf einer Seite des Längsebene angeordnet ist. Die Magnetanordnung erstreckt sich insbesondere nur über einen einseitigen Teilbereich der Umfangsfläche der Linseneinheit. Die Magnetanordnung der Stablinse kann weich- oder permanentmagnetisch ausgebildet sein. Die Magnetanordnung kann zusammenhängend ausgebildet sein oder aus mehreren, voneinander durch Unterbrechungen getrennten Segmenten oder Abschnitten bestehen. Im Rahmen der vorliegenden Anmeldung wird die Einheit oder Baugruppe aus dem stabförmigen Linsenelement bzw. den miteinander verkitteten Linsenelementen und der Magnetanordnung als Stablinse bezeichnet.

Eine erfindungsgemäße Stablinse kann in einem wie oben beschrieben ausgebildeten Optikrohr magnetisch gehalten sein, wodurch in vorteilhafter Weise eine lineare Stablinsenanordnung, die beispielsweise als Bildweiterleiter eines starren Endoskops ausgebildet ist, geschaffen werden kann.

Vorzugsweise ist die Magnetanordnung der Stablinse als mindestens ein parallel zu einer Längsachse der Linseneinheit angeordneter Streifen ausgebildet, der in vorteilhafter Weise im Wesentlichen entlang der gesamten Länge der Linseneinheit verläuft. Der mindestens eine Streifen kann in Längsrichtung unterbrochen sein und mehrere Abschnitte aufweisen. Sind mehrere parallel zueinander verlaufende Streifen vorgesehen, so können diese durch ebenfalls achsenparallel verlaufende Unterbrechungen bzw. Lücken voneinander getrennt sein oder eng aneinander anliegend angeordnet sein. Dadurch, dass die Magnetanordnung als mindestens ein achsenparalleler Streifen ausgebildet ist, ist ein besonders sicheres achsparalleles Halten der Stablinse bzw. der Linseneinheit in einem wie zuvor beschrieben ausgebildeten Optikrohr erreichbar, wobei insbesondere die Längsachsen mehrerer Stablinsen bzw. der Linseneinheiten mehrerer Stablinsen miteinander fluchten.

Die Magnetanordnung der Stablinse ist fest mit der Linseneinheit verbunden. In besonders vorteilhafter Weise ist die Magnetanordnung auf die Umfangsfläche der Stablinse aufgeklebt. Hierfür kann die Magnetanordnung beispielsweise teilzylindrisch ausgebildet sein, wobei der Innendurchmesser der Magnetanordnung geringfügig größer als ein Außendurchmesser einer zylindrisch ausgebildeten Linseneinheit der Stablinse ist, so dass zwischen der Magnetanordnung und der Linseneinheit ein Klebespalt verbleibt, der zum Fixieren der Magnetanordnung zumindest teilweise mit Klebstoff gefüllt wird. Hierdurch wird die Anbringung der Magnetanordnung auf der Linseneinheit weiter erleichtert.

Weiterhin ist es vorteilhaft, dass die Magnetanordnung auf einem hinsichtlich des Durchmessers verjüngten Bereich der Linseneinheit der Stablinse angeordnet ist. Da in einem Bildweiterleiter eines starren Endoskops der radial äußere Bereich eines mittleren Abschnitts der Stablinse für die Bildübertragung nicht benötigt wird, sind derartige Stablinsen häufig mit einem gegenüber den Endbereichen verjüngten mittleren Bereich ausgebildet. Durch die Anordnung der Magnetanordnung in diesem verjüngten Bereich wird eine optimale Ausnutzung des innerhalb des Optikrohrs verfügbaren Bauraums ermöglicht, ohne den für die Bildübertragung benötigten Querschnitt der Linseneinheit der Stablinse wesentlich einzuschränken.

Eine erfindungsgemäße Kombination aus einem Optikrohr und mindestens einer Stablinse umfasst ein Optikrohr, insbesondere ein Optikrohr eines starren Endoskops, wobei das Optikrohr wie oben beschrieben ausgebildet ist, und eine oder mehrere Stablinsen, die wie zuvor beschrieben ausgebildet ist bzw. sind. Eine Kombination aus einem derartigen Optikrohr und mindestens einer derartigen Stablinse ermöglicht auf einfache und sichere Weise die Montage einer Stablinsenanordnung, insbesondere des Bildübertragungssystems eines starren Endoskops, und ein sicheres Halten der mindestens einen Stablinse innerhalb des Optikrohrs.

Sofern in dem Optikrohr eine Mehrzahl von Stablinsen aufgenommen sind, so kann durch die erfindungsgemäße Ausgestaltung des Optikrohrs und der Stablinsen insbesondere erreicht werden, dass die Längsachsen der Stablinsen bzw. der Linseneinheiten der Stablinsen miteinander fluchten und dadurch eine optische Achse der Stablinsenanordnung bilden, wobei eine Dezentrierung und ein Verkippen relativ zur optischen Achse sowie eine Rotation der Stablinsen um ihre Längsachse verhindert werden können. Die Längsachsen der Linseneinheiten der Stablinsen und somit die optische Achse können dabei von der Längsmittelachse des Optikrohrs abweichen. Insbesondere kann eine stabile Kompensation von Abbildungsfehlern des Bildweiterleiters bzw. des gesamten optischen Systems erreicht werden, wobei das optische System zudem besonders unempfindlich gegen toleranzbedingte Abweichungen der Stablinsen ist, beispielsweise gegen Abweichungen von einer nicht-rotationssymmetrischen Form. Insbesondere kann eine besonders hohe Auflösung des optischen Systems gewährleistet werden.

Vorzugsweise ist der Außenradius der mindestens einen Stablinse zumindest im Bereich der Magnetanordnung der Stablinse im Wesentlichen gleich dem Innenradius des Optikrohrs. Der Außenradius der Stablinse kann insbesondere der der Magnetanordnung der Stablinse sein, beispielsweise wenn die Magnetanordnung eine teilzylindrische Form aufweist. Hierdurch wird ein sicheres Halten der Stablinse und eine optimale Ausnutzung des verfügbaren Bauraums innerhalb des Optikrohrs ermöglicht.

Gemäß einer bevorzugten Ausführungsform der Erfindung ist die Magnetanordnung des Optikrohrs permanentmagnetisch ausgebildet, und die Magnetanordnung der Stablinse ist weichmagnetisch ausgebildet. Gemäß einer alternativen bevorzugten Ausführungsform der Erfindung ist die Magnetanordnung des Optikrohrs weichmagnetisch und die der Stablinse permanentmagnetisch ausgebildet. Die permanentmagnetisch ausgebildete Magnetanordnung des Optikrohrs bzw. der Stablinse weist hierfür mindestens einen Permanentmagneten auf. Die Magnetanordnungen des Optikrohrs und der Stablinse können abschnittsweise unterschiedlich aufgebaut sein. Dadurch, dass mindestens eine magnetische Paarung einer permanentmagnetischen Magnetanordnung am Optikrohr mit einer weichmagnetischen Magnetanordnung auf der Stablinse oder umgekehrt vorgesehen ist, wird auf einfache Weise ein sicheres Halten der Stablinse innerhalb des Optikrohrs ermöglicht.

Vorzugsweise ist die permanentmagnetische Magnetanordnung des Optikrohrs bzw. der Stablinse in radialer Richtung magnetisiert. Hierfür kann insbesondere ein Permanentmagnet der betreffenden Magnetanordnung mit radial gerichteter Magnetisierung angeordnet sein. Hierdurch wird ein besonderes sicheres Halten der Stablinse innerhalb des Optikrohrs ermöglicht.

Gemäß einer weiteren bevorzugten Ausführungsform der Erfindung sind sowohl die Magnetanordnung des Optikrohrs als auch die der Stablinse permanentmagnetisch ausgebildet und zur Erzeugung einer magnetischen Anziehungskraft zwischen dem Optikrohr und einer in diesem angeordneten Stablinse magnetisiert. Dies bedeutet, dass unterschiedliche Pole der permanentmagnetischen Magnetanordnungen des Optikrohrs und der Stablinse zueinander gerichtet sind. Auch durch eine solche magnetische Paarung wird ein besonderes sicheres Halten der Stablinse innerhalb des Optikrohrs ermöglicht.

Zur Montage einer oder mehrerer erfindungsgemäßen Stablinsen in einem erfindungsgemäßen Optikrohr eines Endoskops werden zunächst die jeweiligen Magnetanordnungen einseitig auf die Linseneinheiten der Stablinsen sowie auf das Optikrohr aufgebracht, beispielsweise aufgeklebt. Dabei ist zumindest eine magnetische Paarung zwischen den Stablinsen und dem Optikrohr im oben genannten Sinne notwendig. Sodann werden die Stablinsen, die jeweils als vormontierte Baugruppe eine stabförmige Linseneinheit und eine auf dieser befestigte Magnetanordnung umfassen, in der durch die Funktion des optischen Systems vorgegebenen Anordnung und Ausrichtung, ggf. mit weiteren optischen Elementen wie etwa Abstandshaltern und Blenden, in das derart vormontierte Optikrohr eingeschoben. Aufgrund der einseitigen Anordnung der Magnetanordnungen werden die Stablinsen miteinander fluchtend auf eine Seite des Optikrohrs angezogen und durch die Magnetkraft sowie aufgrund der durch die Magnetkraft verstärkten Reibung zwischen den Stablinsen und der Innenseite des Optikrohrs gehalten. In dieser Anordnung werden die Stablinsen auch beim Gebrauch des Endoskops gehalten und sind hierdurch auch gegen eine Rotation um ihre Längsachse fixiert. Zu Reparaturzwecken ist jedoch weiterhin eine Entnahme der Stablinsen aus dem Optikrohr möglich.

Ein erfindungsgemäßes Endoskop weist ein langerstrecktes Optikrohr auf, das innerhalb eines lang erstreckten Endoskopschafts angeordnet ist und auch in einen Kopf des Endoskops hineinreichen kann. Der Endoskopschaft ist als starrer Endoskopschaft ausgebildet, wobei eine gewisse Biegung zulässig sein kann. Das Optikrohr kann beispielsweise als Innenrohr einer Endoskopoptik, die hier ebenfalls als Endoskop bezeichnet wird, oder als Systemrohr eines Endoskops, das beispielsweise Arbeitskanäle für endoskopische Instrumente oder weitere Kanäle umfassen kann, ausgebildet sein. Innerhalb des Optikrohrs des Endoskops ist mindestens eine Stablinse, vorzugsweise eine Mehrzahl von Stablinsen angeordnet, wobei das Optikrohr und die mindestens eine Stablinse sowie die Kombination aus dem Optikrohr und der mindestens einen Stablinse wie oben beschrieben ausgebildet sind.

Die eine oder mehreren Stablinsen sind in dem Optikrohr durch magnetische Kräfte einseitig in Berührung mit der Wandung des Optikrohrs gehalten. In Längsrichtung sind die Stablinse bzw. die Stablinsen durch Reibungskräfte zwischen den einander berührenden Oberflächen der mindestens einen Stablinse und des Optikrohrs sowie ggf. durch eine von einer proximalseitig angeordneten Systemfeder auf die Anordnung der Stablinsen und ggf. weiterer optischer Elemente ausgeübte Vorspannung fixiert. Durch die von der einseitigen, sich achsenparallel erstrechenden Magnetanordnung des Optikrohrs ausgeübten magnetischen Anziehungskräfte wird ein Verkippen verhindert und, sofern mehrere Stablinsen vorgesehen sind, diese in einer miteinander fluchtenden Anordnung gehalten. Ferner werden durch die Reibungskräfte sowie durch die einseitige angebrachten Magnetanordnungen des Optikrohrs und der Stablinsen auch eine Bewegung der mindestens einen Stablinse in Umfangsrichtung des Optikrohrs sowie eine Rotation der Stablinse um ihre Längsachse verhindert. Durch die erfindungsgemäße Ausbildung des Optikrohrs und der in diesem aufgenommenen Stablinsen kann insbesondere eine Dezentrierung, ein Verkippen und eine Rotation der Stablinsen um ihre Längsachse verhindert werden und eine stabile Kompensation von Abbildungsfehlern auch beim Betrieb des Endoskops gewährleistet werden, wodurch beispielsweise eine besonders hohe Auflösung des optischen Systems ermöglicht werden kann.

Es versteht sich, dass die vorstehend genannten und die nachstehend noch zu erläuternden Merkmale nicht nur in der jeweils angegebenen Merkmalskombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung bevorzugter Ausführungsbeispiele und der beigefügten Zeichnung. Es zeigen:
Fig. 1 ein Optikrohr und eine Stablinse gemäß einem ersten Ausführungsbeispiel der Erfindung in einem schematischen Querschnitt;
Fig. 2a und 2b das Optikrohr mit zwei darin angeordneten Stablinsen gemäß dem ersten Ausführungsbeispiel in einem schematischen Längsschnitt, wobei Fig. 2b den in Fig. 2a mit einem Kreis markierten Ausschnitt in einer vergrößerten Darstellung zeigt;
Fig. 3 das Optikrohr gemäß dem ersten Ausführungsbeispiel in einer perspektivischen Ansicht;
Fig. 4 ein Optikrohr und eine Stablinse gemäß einem zweiten Ausführungsbeispiel der Erfindung in einem schematischen Querschnitt;
Fig. 5 das Optikrohr gemäß dem zweiten Ausführungsbeispiel in einer perspektivischen Ansicht;
Fig. 6 ein Endoskop in einer teilweise aufgebrochenen Seitenansicht.

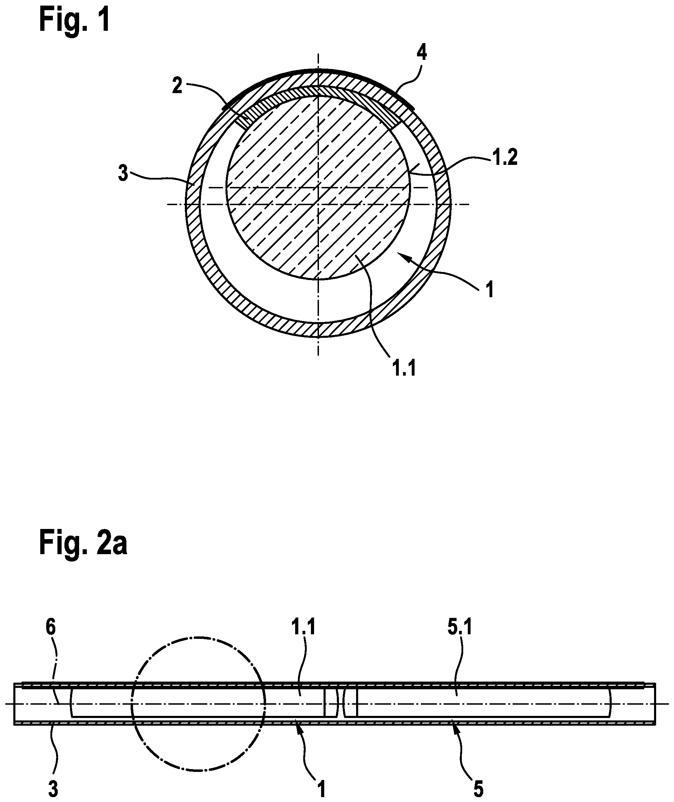

Wie in Fig. 1 gezeigt, ist gemäß einem ersten Ausführungsbeispiel der Erfindung auf die Mantelfläche 1.2 der zylindrischen Linseneinheit 1.1 einer Stablinse 1 einseitig eine Magnetanordnung (Magnet 2) aufgeklebt. Das Optikrohr 3 weist ebenfalls einseitig eine aufgeklebte Magnetanordnung (Magnetstreifen 4) auf. Der Magnetstreifen 4 nimmt etwa ein Viertel des Umfangs des Optikrohrs 3 ein, d.h. gesehen von einer Längsmittelachse des Optikrohrs 3 ca. 90°. Der Magnet 2 liegt an der Innenseite des Optikrohrs 3 im Bereich des Magnetstreifens 4 an und nimmt etwa ebenfalls ein Viertel des Umfangs der Linseneinheit 1.1 ein, aufgrund des gegenüber dem Innendurchmesser des Optikrohr 3 etwas geringeren Außendurchmessers der Linseneinheit 1.1 etwas mehr als ein Vierteil bzw. 90°. Während der Magnetstreifen 4 in dem in Fig. 1 gezeigten Ausführungsbeispiel als Schicht bzw. als flexibler Streifen ausgebildet ist, die bzw. der sich an die äußere Umfangsfläche des Optikrohrs 3 anlegt, weist der Magnet 2 einen Innenradius auf, der im Wesentlichen dem Außenradius der Linseneinheit 1.1 der Stablinse 1 entspricht und einen Außenradius, der dem Innenradius des Optikrohrs 3 entspricht. Dabei kann ein Klebespalt zwischen dem Magnet 2 und der Linseneinheit 1.1, der mit Klebstoff gefüllt ist, vorgesehen sein; auch der Magnetstreifen 4 ist durch Kleben befestigt.

Um die Stablinse 1 fest im Optikrohr 3 zu halten, sind der Magnet 2 und der Magnetstreifen 4 in der Weise magnetisch miteinander gepaart, dass entweder der Magnet 2 permanentmagnetisch und der Magnetstreifen 4 weichmagnetisch oder der Magnet 2 weichmagnetisch und der Magnetstreifen 4 permanentmagnetisch ausgebildet ist, oder sowohl der Magnet 2 als auch der Magnetstreifen 4 sind permanentmagnetisch mit zueinander gerichteten ungleichen Polen ausgebildet. Hierdurch wird eine Anziehungskraft zwischen dem Magnetstreifen 4 und dem Magnet 2 erzeugt, wobei das Optikrohr 3 ausreichend dünnwandig oder nicht-ferromagnetisch ausgebildet ist, um eine entsprechende magnetische Wirkung durch das Optikrohr 3 hindurch zu ermöglichen. Wie in Fig. 1 zu erkennen ist, ist die Linseneinheit 1.1 der Stablinse 1 asymmetrisch innerhalb des Optikrohrs 3 angeordnet, so dass die Symmetrieachse der Linseneinheit 1.1 gegenüber der Längsmittelachse des Optikrohrs 3 versetzt ist.

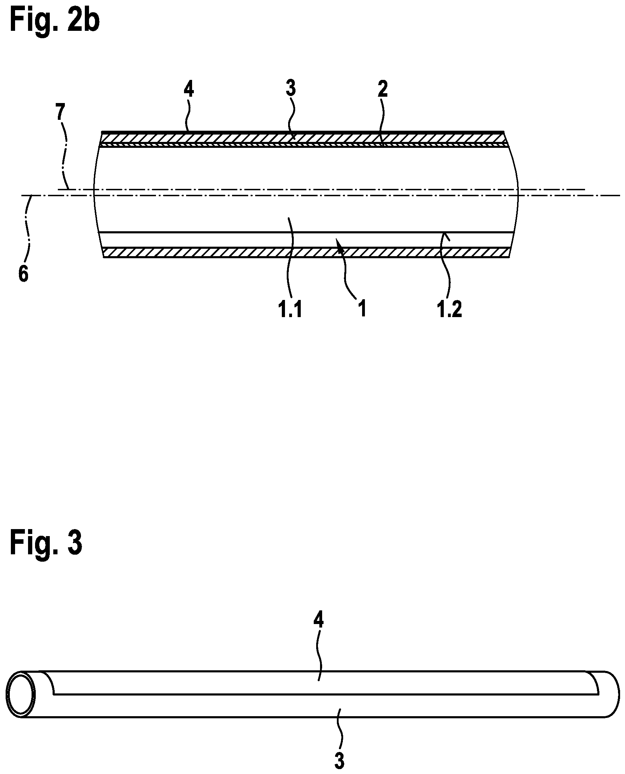

Dies wird in Fig. 2a und 2b deutlich, worin die in Fig. 1 im Querschnitt gezeigte Anordnung im Längsschnitt dargestellt ist. Innerhalb des Optikrohrs 3 sind gemäß Fig. 2a zwei Stablinsen 1, 5 angeordnet, die jeweils eine zylindrische Linseneinheit 1.1, 5.1 umfassen, die jeweils aus einem länglichen Stabteil und einer mit diesem verkitteten dünnen Linse besteht. Die Linseneinheiten 1.1, 5.1 sind mit den dünnen Linsen einander zugewandt und bilden ein Umkehrsystem. Das Optikrohr 3 kann weitere derartige Umkehrsysteme aufweisen (nicht dargestellt).

Wie in Fig. 2a angedeutet und in Fig. 2b ausschnittsweise vergrößert dargestellt, wird die Stablinse 1 einseitig an der Innenwand des Optikrohrs 3 gehalten, wobei die Längsachse 7 der Stablinse 1, die die Symmetrieachse der Linseneinheit 1.1 ist, gegenüber der Längsachse 6 des Optikrohrs 3, die die Längsmittelachse des Optikrohrs 3 ist, versetzt ist. Der einseitig auf der Außenseite des Optikrohrs 3 angeordnete Magnetstreifen 4 und der einseitig auf der Linseneinheit 1.1 der Stablinse 1 aufgeklebte Magnet 2 üben eine magnetische Anziehungskraft aufeinander aus, so dass die Stablinse 1 fest an der Innenwand des Optikrohrs 3 gehalten wird. Der Magnet 2 erstreckt sich achsenparallel im Wesentlichen über die gesamte Länge der Linseneinheit 1.1. Die Stablinse 1 und das Optikrohr 3 sind zylindrisch ausgebildet, und die Stablinse 1 wird achsenparallel innerhalb des Optikrohrs 3 gehalten. In gleicher Weise ist die Stablinse 5 innerhalb des Optikrohrs gehalten und dabei in der gleichen Richtung wie die Stablinse 1 mit ihrer Längsachse gegenüber der Längsachse 6 des Optikrohrs versetzt. Die Symmetrie- bzw. Längsachse der Linseneinheit 5.1 fluchtet somit mit der Symmetrie- bzw. Längsachse 7 der Linseneinheit 1.1 und bildet die optische Achse der Stablinsenanordnung, die durch die Stablinsen 1, 5 gebildet wird. Hierdurch wird eine besonders toleranzstabile Anordnung der Stablinsen 1, 5 erreicht. Insbesondere kann eine Rotation der Stablinsen 1, 5 um ihre Längsachsen vermieden werden, ohne dass eine feste Verbindung zwischen den Stablinsen 1, 5 und dem Optikrohr 3 notwendig wäre. Diese können daher in einfacher Weise in das Optikrohr 3 eingeschoben und gegebenenfalls zu einem späteren Zeitpunkt zu Reparaturzwecken wieder aus diesem entnommen werden.

In Fig. 3 ist das Optikrohr 3 gemäß dem ersten Ausführungsbeispiel der Erfindung in einer perspektivischen Ansicht gezeigt. Wie in Fig. 3 zu erkennen ist, erstreckt sich der Magnetstreifen 4 in Längsrichtung des Optikrohrs 3 im Wesentlichen über die gesamte Länge des Optikrohrs 3.

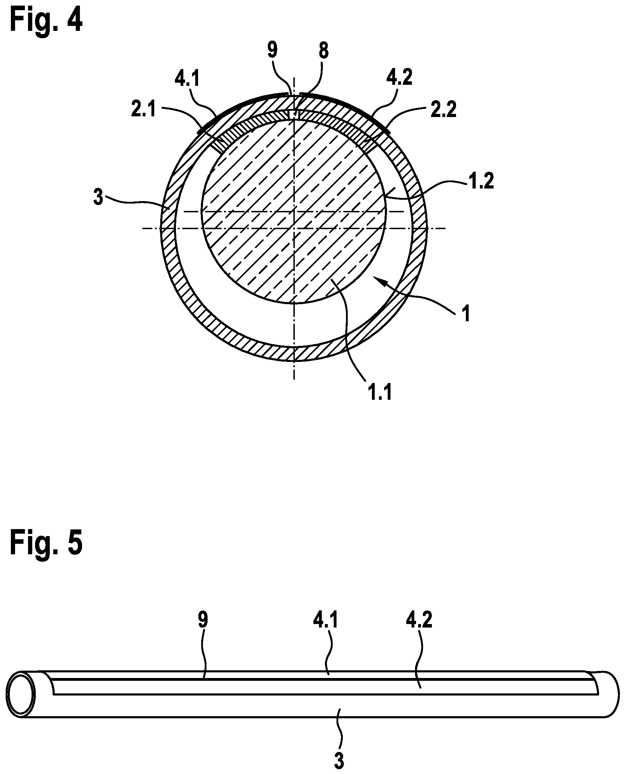

Gemäß einer zweiten Ausführungsform der Erfindung, die in den Figuren 4 und 5 dargestellt ist, besteht die Magnetanordnung der Stablinse 1 aus zwei Segmenten 2.1., 2.2, die durch eine in Längsrichtung der Stablinse 1 verlaufende Lücke 8 voneinander getrennt sind. In ähnlicher Weise besteht die Magnetanordnung des Optikrohrs 3 aus zwei Teilstreifen 4.1, 4.2, die beide in Längsrichtung des Optikrohrs 3 verlaufen und durch eine Lücke 9 voneinander getrennt sind. Im Übrigen ist das in den Figuren 4 und 5 dargestellte Ausführungsbeispiel wie das in den Figuren 1 bis 3 gezeigte aufgebaut.

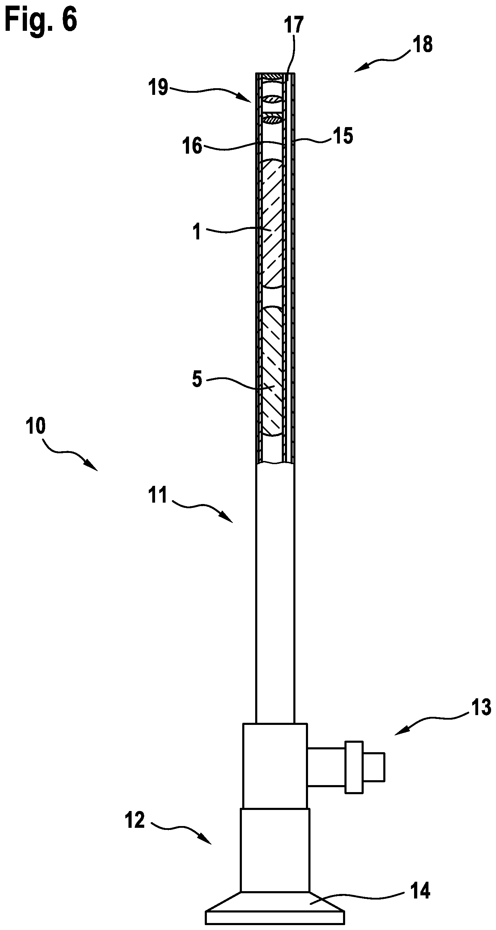

In Fig. 6 ist beispielhaft ein starres Endoskop 10 mit Stablinsen gemäß den vorstehend beschriebenen Ausführungsbeispielen schematisch dargestellt. Das Endoskop 10 umfasst einen langerstreckten, zur Einführung in einen körperinneren Hohlraum ausgebildeten Endoskopschaft 11 und einen am proximalen Ende des Endoskopschafts 11 angeordneten Endoskopkopf 12, der einen Lichtanschluss 13 und ein Okular mit einer Okularmuschel 14 aufweist. Innerhalb des Außenrohrs 15 des Endoskopschafts 11 sind das Optikrohr 16 sowie Lichtleiter 17 zur Übertragung des am Lichtanschluss 13 eingekoppelten Beleuchtungslichts zum distalen Endbereich 18 des Endoskops 10 angeordnet. Im Innenraum des Optikrohrs 16 sind eine Objektivlinsenanordnung 19 sowie Stablinsen 1, 5, die zur Weiterleitung des von der Objektivlinsenanordnung 19 entworfenen endoskopischen Bilds vom distalen Endbereich 18 zum proximalen Endbereich des Endoskops 10 dienen, aufgenommen. Das Optikrohr 16 sowie die Stablinsen 1, 5 sind wie zu Fig. 1 bis 5 beschrieben aufgebaut und in Fig. 6 lediglich symbolisch dargestellt.
Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Stablinse
- 1.1: Linseneinheit
- 1.2: Mantelfläche
- 2: Magnet
- 2.1: Segment
- 2.2: Segment
- 3: Optikrohr
- 4: Magnetstreifen
- 4.1: Teilstreifen
- 4.2: Teilstreifen
- 5: Stablinse
- 5.1: Linseneinheit
- 6: Längsachse
- 7: Längsachse
- 10: Endoskop
- 11: Endoskopschaft
- 12: Endoskopkopf
- 13: Lichtanschluss
- 14: Okularmuschel
- 15: Außenrohr
- 16: Optikrohr
- 17: Lichtleiter
- 18: Distaler Endbereich
- 19: Objektivlinsenanordnung

## Patentansprüche

1. Stablinse, umfassend eine stabförmige Linseneinheit (1.1, 5.1) aus einem stabförmigen Linsenelement oder mehreren miteinander verkitteten Linsenelementen, **dadurch gekennzeichnet, dass** die Linseneinheit (1.1, 5.1) auf ihrer Umfangsfläche einseitig eine Magnetanordnung (2) aufweist, wobei die Magnetanordnung als mindestens ein parallel zu einer Längsachse (7) der Linseneinheit (1.1, 5.1) gerichteter Streifen ausgebildet ist.

2. Stablinse nach Anspruch 1, **dadurch gekennzeichnet, dass** die Magnetanordnung (2) auf die Umfangsfläche aufgeklebt ist.

3. Stablinse nach einem der Ansprüche 1 oder 2, **dadurch gekennzeichnet, dass** die Magnetanordnung (2) in einem gegenüber einem Endabschnitt verjüngten mittleren Abschnitt der Linseneinheit (1.1, 5.1) angeordnet ist.

4. Kombination aus mindestens einer Stablinse (1, 5) gemäß einem der Ansprüche 1 bis 3 und einem Optikrohr (3) für ein Endoskop (10), das einseitig eine sich parallel zu einer Längsachse (6) des Optikrohrs (3) erstreckende Magnetanordnung (4) aufweist, wobei die Magnetanordnung (4) des Optikrohrs (3) als mindestens ein zur Längsachse (6) des Optikrohrs (3) paralleler, sich im Wesentlichen über die gesamte Länge des Optikrohrs (3) erstreckender Streifen ausgebildet ist, so dass die Magnetanordnung (4) des Optikrohrs (3) und die Magnetanordnung (2) der Stablinse (1,5) eine magnetische Anziehungskraft aufeinander ausüben, so dass die Stablinse (1,5) im Optikrohr (3) gehalten wird.

5. Kombination nach Anspruch 4, **dadurch gekennzeichnet, dass** ein Außenradius der mindestens einen Stablinse (1, 5) im Bereich der Magnetanordnung (2) der Stablinse (1, 5) gleich einem Innenradius des Optikrohrs (3) ist.

6. Kombination nach Anspruch 4 oder 5, **dadurch gekennzeichnet, dass** entweder die Magnetanordnung (4) des Optikrohrs (3) permanentmagnetisch und die Magnetanordnung (2) der Stablinse (1, 5) weichmagnetisch ausgebildet ist oder die Magnetanordnung (4) des Optikrohrs (3) weichmagnetisch und die Magnetanordnung (2) der Stablinse (1, 5) permanentmagnetisch ausgebildet ist.

7. Kombination nach Anspruch 6, **dadurch gekennzeichnet, dass** die permanentmagnetisch ausgebildete Magnetanordnung (4) des Optikrohrs (3) bzw. der Stablinse (1, 5) radial magnetisiert ist.

8. Kombination nach Anspruch 6 oder 7, **dadurch gekennzeichnet, dass** die Magnetanordnung (4) des Optikrohrs (3) und die Magnetanordnung (2) der Stablinse (1, 5) permanentmagnetisch ausgebildet und zur Erzeugung einer magnetischen Anziehungskraft zwischen dem Optikrohr (3) und der Stablinse (1, 5) magnetisiert sind.

9. Kombination nach einem der Ansprüche 1 bis 8, **dadurch gekennzeichnet, dass** die Magnetanordnung (4) auf einer Außenseite des Optikrohrs (3) angeordnet ist.

10. Kombination nach Anspruch 9, **dadurch gekennzeichnet, dass** die Magnetanordnung auf die Außenseite des Optikrohrs (3) aufgeklebt ist.

11. Endoskop mit einem Optikrohr (3) und mindestens einer in dem Optikrohr (3) aufgenommenen Stablinse (1, 5), **dadurch gekennzeichnet, dass** das Optikrohr (3) und die Stablinse (1, 5) eine Kombination gemäß einem der Ansprüche 4 bis 10 bilden.

## Claims

1. Rod lens comprising a rod-shaped lens unit (1.1, 5.1) made of a rod-shaped lens element or a plurality of lens elements cemented together, **characterized in that** the lens unit (1.1, 5.1) has, on the circumferential surface thereof, a magnet arrangement (2) on one side, wherein the magnet arrangement is embodied as at least one strip directed parallel to a longitudinal axis (7) of the lens unit (1.1, 5.1).

2. Rod lens according to Claim 1, **characterized in that** the magnet arrangement (2) is adhesively bonded onto the circumferential surface.

3. Rod lens according to either of Claims 1 and 2, **characterized in that** the magnet arrangement (2) is arranged in a central portion of the lens unit (1.1, 5.1) which is tapered in relation to an end portion.

4. Combination of at least one rod lens (1, 5) according to one of Claims 1 to 3 and an optics tube (3) for an endoscope (10), which, on one side, has a magnet arrangement (4) extending parallel to a longitudinal axis (6) of the optics tube (3), wherein the magnet arrangement (4) of the optics tube (3) is embodied as at least one strip extending parallel to the longitudinal axis (6) of the optics tube (3) substantially over the whole length of the optics tube (3) such that the magnet arrangement (4) of the optics tube (3) and the magnet arrangement (2) of the rod lens (1, 5) exert an attractive magnetic force on one another such that the rod lens (1, 5), is held in the optics tube (3).

5. Combination according to Claim 4, **characterized in that** an external radius of the at least one rod lens (1, 5) in the region of the magnet arrangement (2) of the rod lens (1, 5) is equal to an internal radius of the optics tube (3).

6. Combination according to Claim 4 or 5, **characterized in that** either the magnet arrangement (4) of the optics tube (3) has a permanent magnetic embodiment and the magnet arrangement (2) of the rod lens (1, 5) has a soft magnetic embodiment or the magnet arrangement (4) of the optics tube (3) has a soft magnetic embodiment and the magnet arrangement (2) of the rod lens (1, 5) has a permanent magnetic embodiment.

7. Combination according to Claim 6, **characterized in that** the magnet arrangement (4), with the permanent magnetic embodiment, of the optics tube (3) or of the rod lens (1, 5) has a radial magnetization.

8. Combination according to Claim 6 or 7, **characterized in that** the magnet arrangement (4) of the optics tube (3) and the magnet arrangement (2) of the rod lens (1, 5) have a permanent magnetic embodiment and are magnetized for generating an attractive magnetic force between the optics tube (3) and the rod lens (1, 5) .

9. Combination according to one of Claims 1 to 8, **characterized in that** the magnet arrangement (4) is arranged on an outer side of the optics tube (3).

10. Combination according to Claim 9, **characterized in that** the magnet arrangement is adhesively bonded onto the outer side of the optics tube (3).

11. Endoscope comprising an optics tube (3) and at least one rod lens (1, 5) received in the optics tube (3), **characterized in that** the optics tube (3) and the rod lens (1, 5) form a combination according to one of Claims 4 to 10.

## Revendications

1. Lentille en barreau, comprenant une unité de lentille (1.1, 5.1) en forme de barreau constituée d'un élément de lentille en forme de barreau ou de plusieurs éléments de lentille en forme de barreau agglomérés, **caractérisée en ce que** l'unité de lentille (1.1, 5.1) possède d'un côté sur sa surface périphérique un arrangement d'aimants (2), l'arrangement d'aimants (2) étant réalisé sous la forme d'au moins une bande dirigée parallèlement à un axe longitudinal (7) de l'unité de lentille (1.1, 5.1).

2. Lentille en barreau selon la revendication 1, **caractérisée en ce que** l'arrangement d'aimants (2) est collé sur la surface périphérique.

3. Lentille en barreau selon l'une des revendications 1 ou 2, **caractérisée en ce que** l'arrangement d'aimants (2) est disposé dans une portion centrale de l'unité de lentille (1.1, 5.1) amincie par rapport à une portion d'extrémité.

4. Combinaison d'au moins une lentille en barreau (1, 5) selon l'une des revendications 1 à 3 et d'un tube optique (3) pour un endoscope (10), lequel possède d'un côté un arrangement d'aimants (4) qui s'étend parallèlement à un axe longitudinal (6) du tube optique (3), l'arrangement d'aimants (4) du tube optique (3) étant réalisé sous la forme d'au moins une bande parallèle à l'axe longitudinal (6) du tube optique (3) qui s'étend pour l'essentiel sur toute la longueur du tube optique (3), de sorte que l'arrangement d'aimants (4) du tube optique (3) et l'arrangement d'aimants (2) de la lentille en barreau (1, 5) exercent l'un sur l'autre une force d'attraction magnétique, de sorte que la lentille en barreau (1, 5), est maintenue dans le tube optique (3).

5. Combinaison selon la revendication 4, **caractérisée en ce qu'**un rayon extérieur de l'au moins une lentille en barreau (1, 5) dans la zone de l'arrangement d'aimants (2) de la lentille en barreau (1, 5) est égal à un rayon intérieur du tube optique (3).

6. Combinaison selon la revendication 4 ou 5, **caractérisée en ce que** l'arrangement d'aimants (4) du tube optique (3) est réalisé à magnétisme permanent et l'arrangement d'aimants (2) de la lentille en barreau (1, 5) à magnétisme doux, ou l'arrangement d'aimants (4) du tube optique (3) est réalisé à magnétisme doux et l'arrangement d'aimants (2) de la lentille en barreau (1, 5) à magnétisme permanent.

7. Combinaison selon la revendication 4 ou 5, **caractérisée en ce que** l'arrangement d'aimants (4) réalisé à magnétisme permanent du tube optique (3) ou de la lentille en barreau (1, 5) est magnétisé dans le sens radial.

8. Combinaison selon la revendication 6 ou 7, **caractérisée en ce que** l'arrangement d'aimants (4) du tube optique (3) et l'arrangement d'aimants (2) de la lentille en barreau (1, 5) sont réalisés à magnétisme permanent et sont magnétisés pour générer une force d'attraction magnétique entre le tube optique (3) et la lentille en barreau (1, 5).

9. Combinaison selon l'une des revendications 1 à 8, **caractérisée en ce que** l'arrangement d'aimants (4) est disposé sur un côté extérieur du tube optique (3).

10. Combinaison selon la revendication 9, **caractérisée en ce que** l'arrangement d'aimants (4) est collé sur un côté extérieur du tube optique (3).

11. Endoscope comprenant un tube optique (3) et au moins une lentille en barreau (1, 5) accueillie dans le tube optique (3), **caractérisé en ce que** le tube optique (3) et la lentille en barreau (1, 5) forment une combinaison selon l'une des revendications 4 à 10.
